# EUROPÄISCHE PATENTSCHRIFT

(11) **EP 1 287 812 B1**
(45) Veröffentlichungstag und Bekanntmachung des Hinweises auf die Patenterteilung: **05.04.2006**
(21) Anmeldenummer: 02013122.3
(22) Anmeldetag: 14.06.2002
(51) Int. Cl.: A61Q 5/04, A61Q 5/10, A61K 8/41, A61K 8/49, A61K 8/44, A61K 8/46

(54) **Verfahren zum gleichzeitigen Färben und dauerhaften Verformen von Haaren**
Method of simultaneously dyeing and permanent waving the hair
Procédé de mise en forme et de coloration simultanées des cheveux

(30) Priorität: 04.09.2001 DE 10143293
(43) Veröffentlichungstag der Anmeldung: 05.03.2003
(73) Patentinhaber: Wella Aktiengesellschaft, 64274 Darmstadt (DE)
(72) Erfinder: Schonert, Dieter, 64354 Reinheim-Georgenhausen (DE); Schmidt-Hörr, Anette, 64401 Gr.-Bieberau (DE); Lenz, Uwe, Dr., 64673 Zwingenberg (DE)

(56) Entgegenhaltungen:
- EP-A- 0 352 375
- DE-A- 19 810 887
- GB-A- 951 021
- US-A- 3 399 682
- US-A- 3 654 936
- US-A- 4 630 621
- KOJIMA H, TAKENAKA J (NISHIRENJI TRADING K.K.): "Hair preparations containing reducing agents, sequestering agents, metallic salts, dyes and oxidizing agents" CHEMICAL ABSTRACTS + INDEXES, Bd. 107, Nr. 20, 16. November 1987 (1987-11-16), XP002183446 AMERICAN CHEMICAL SOCIETY. COLUMBUS, US ISSN: 0009-2258

## Beschreibung

Gegenstand der Erfindung ist ein Verfahren zur dauerhaften Verformung von Haaren, insbesondere von Naturhaar und gefärbtem Haar, bei gleichzeitiger Färbung bzw. Farbauffrischung (Farbemeuerung, Farbwiederherstellung). Im 1. Schritt wird das Haar gequollen und in seinem natürlichen pH-Wert verändert; gleichzeitig werden die Disulfidbindungen des Haares geöffnet und die Farbstoffe im Haar eingelagert. Im 2. Schritt werden die Schwefelbrücken nach erfolgter Umformung geschlossen und die Farbstoffe im Haar oxidativ entwickelt.

Die klassische Technik zur Durchführung der dauerhaften Haarverformung besteht darin, daß in einer ersten Stufe die Disulfid-Bindungen des Haarkeratins mit Hilfe eines Mittels, welches einen reduzierenden Wirkstoff enthält (Verformungsmittel), geöffnet werden, sodann das Haar in die gewünschte Form gebracht wird und anschließend die Disulfid-Bindungen unter Verwendung eines einen oxidierenden Wirkstoff enthaltenden Mittels (Fixiermittel) wieder verknüpft werden. Als reduzierende Wirkstoffe werden hierbei insbesondere Sulfite, Thioglykolsäure, Thiomilchsäure, 3-Mercapto-propionsäure, Mercaptocarbonsäureester und Cystein verwendet. Diese Mittel sind entweder sauer (Sulfit, Bisulfit und Mercaptocarbonsäureester) oder alkalisch (Alkali- und Ammoniumsalze von Mercaptocarbonsäuren) eingestellt. Im Falle von alkalisch eingestellten Verformungsmittein wird die erforderliche Alkalität vor allem durch Zusatz von Ammoniak, organischen Aminen, Ammonium- oder Alkalicarbonat und Ammonium- oder Alkalihydrogencarbonat erreicht. Als Fixiermittel werden insbesondere wasserstoffperoxidhaltige oder bromathaltige Flüssigkeiten verwendet.

Die Durchführung der dauerhaften Verformung von menschlichen Haaren erfolgt im allgemeinen, indem man das gewaschene und handtuchtrockene Haar zunächst in mehrere Partien aufteilt und diese Partien sodann auf Wickler wickelt.

Nach Beendigung des Wickelvorganges werden die Wickler mit der erforderlichen Menge des Dauerverformungsmittels gründlich durchfeuchtet. Die für eine Dauerwellung verwendeten Wickler haben einen Durchmesser von etwa 5 bis 13 Millimetern, während für eine Haarentkräuselung Wickler mit einem Durchmesser von über 13 Millimetern erforderlich sind.

Die Einwirkungszeit des Verformungsmittels auf das Haar beträgt, bei der Dauerwellung, je nach Haarbeschaffenheit und dem gewünschten Grad der Umformung etwa 3 bis 30 Minuten. Durch Wärmezufuhr, beispielsweise unter Verwendung eines Wärmestrahlers oder einer Trockenhaube, lässt sich diese Einwirkungszeit verkürzen.

Nach Ablauf der erforderlichen Einwirkungszeit des Verformungsmittels wird das Haar mit Wasser gespült und mit einem Fixiermittel, zum Beispiel einer wässrigen Lösung von Hydrogenperoxid oder Kaliumbromat, behandelt. Die Einwirkungszeit des Fixiermittels beträgt hierbei üblicherweise etwa 1 bis 30 Minuten. Anschließend werden die Wickler entfernt, gegebenenfalls das Haar nochmals einige Minuten lang mit dem Fixiermittel nachbehandelt und sodann gründlich mit Wasser ausgespült, zur Frisur gelegt und getrocknet.

Häufig wird aber nicht nur eine Haarverformung, sondern zusätzlich auch eine Färbung oder Tönung des Haares gewünscht. Insbesondere bei der Haarumformung von gefärbtem Haar verliert das Haar einen großen Teil seiner Farbintensität und seines Glanzes. Hierbei ist es dann erforderlich, nach der Verformungsbehandlung eine gesonderte Färbebehandlung durchzuführen. Im Falle einer oxidativen Färbung führt dies zu einer übermäßigen Beanspruchung des Haares, da jede oxidative Färbung oder Haarverformung einen schwerwiegenden Eingriff in die Haarstruktur darstellt. Bei Verwendung einer Tönung ist das Ergebnis, bedingt durch die unterschiedlichen Strukturzonen, über die Haarlänge ungleichmäßig und die Haltbarkeit ist nur für kurze Zeit gewährleistet, da es zum Auswaschen der Farbe nach wenigen Haarwäschen kommt. Weiterhin führt dies zu einer Verlängerung des gesamten Behandlungsablaufes.

Eine Verringerung dieser Strukturbeanspruchung der Haare sowie eine Verbesserung der Haltbarkeit der Farbe kann durch eine gleichzeitige Durchführung von Haarverformungs- und Färbebehandlung erreicht werden. Eine derartige Kombination der beiden Behandlungen ist zudem auch zeitsparend.

Aus diesem Grunde wurde bereits mehrfach versucht, die Verformung und Färbung von Haaren in einem Arbeitsgang durchzuführen. So ist zum Beispiel aus der DE-AS 1 129 261 sowie der GB-PS 876 663 ein Verfahren bekannt, das eine gleichzeitige Dauerwellung und Färbung von Haaren, unter anderem auch von weißem und grauem Haar, ermöglicht. Dieses Verfahren basiert auf einem Verformungsmittel, welches aus einer wässrigen Lösung eines keratinreduzierenden Wirkstoffes und eines geeigneten basischen Farbstoffes besteht. Die verwendeten Farbstoffe, wie zum Beispiel Kristallviolett, Methylenblau, Fuchsin oder Malachitgrün, liegen in Form der stabileren Leukoverbindungen vor und werden erst bei der anschließenden oxidativen Fixierung in den eigentlichen Farbstoff umgewandelt.

DE-AS 19 810 887 stellt ein Verfahren zum gleichzeitigen Dauerwellen und Färben vor, bei dem Farbstoffe vom Typ der reaktiven Carbonyl verbindung eingesetzt werden. Zur Intensivierung der Färbung sind Verbindungen mit primärer oder sekundärer Amino- oder Hydroxygruppe enthalten.

In dem Übersichtsartikel "Dauerwellen und Haarfärben in einem Arbeitsgang" von R. Heilingötter, Kosmetik-Parfüm-Drogen Rundschau 3/4 (1965), Seiten 35 und 36, wird die Möglichkeit einer gleichzeitigen Tönung und Verformung der Haare durch Zusatz von Oxidationsfarbstoffen (in Form ihrer Vorstufen) zu einer alkalischen Thioglykolatlösung diskutiert. Schließlich ist aus der EP-B 0 352 375 ein Verfahren zur dauerhaften Verformung und gleichzeitigen Färbung von Haaren bekannt, bei dem das Haar zunächst 8 bis 20 Minuten lang mit einem ersten Mittel, enthaltend als keratinreduzierenden Wirkstoffe 0,1 bis 6 Gew.% Thioglykolat und 3 bis 10 Gew.% Cystein sowie als Oxidationsfarbstoffe 0,01 bis 4 Gew.% para- und/oder ortho-Phenylendiamin, 0,01 bis 1 Gew.% Resorcin, ferner 0,01 bis 1 Gew.% Antioxidans, 0,01 bis 1 Gew.% Schwermetall-Komplexbildner, 0,01 bis 1 Gew.% Tensid und Alkalisierungsmittel zur Einstellung eines pH von 9,0 bis 9,5, behandelt wird und danach ohne vorheriges Ausspülen ein zweites Mittel mit 3 bis 8 Gew.% Wasserstoffperoxid aufgebracht wird.

Alte bisher beschriebenen Verfahren zur dauerhaften Verformung und gleichzeitigen Färbung oder Tönung der Haare weisen jedoch Nachteile auf, so dass die erzielten Ergebnisse nicht immer zufriedenstellend sind. So sind einige der verwendeten Farbstoffe, wie beispielsweise bestimmte Oxidationshaarfarbstoffe, gegenüber der stark reduzierend wirkenden, alkalischen Thioglykolatiösung unbeständig. Sie werden bei längerer Lagerung reduktiv angegriffen und lassen in ihrer Färbekraft nach, so dass die Herstellung gebrauchsfertiger Zubereitungen unmöglich ist. Zudem sind die besonders interessanten Rot-Töne meist mit diesen Farbstoffen nicht erhältlich. Andere zur gleichzeitigen Haarfärbung bei der Verformungsbehandlung verwendeten Farbstoffe, wie beispielsweise die in der DE-AS 1 129 261 und derGB-PS 876 663 beschriebenen Farbstoffe, besitzen nur eine ungenügende Lichtechtheit.

Herkömmliche Dauerwellverfahren führen besonders bei gefärbtem Haar zu einem starken Farbverlust. Dauergewelltes Naturhaar verliert merkbar an Glanz und wird durch die Behandlung teilweise aufgehellt. Dadurch wirkt es stumpf und kraftlos.

Aufgabe der vorliegenden Erfindung ist es daher, ein Verfahren zur Verfügung zu stellen, das es besser, schneller und schonender als bisher ermöglicht, mehschliche Haare, insbesondere Naturhaar, zu Färben und gefärbtem Haar seine Farbintensität zu bewahren bzw. es aufzufrischen (emeuem) und seinen Glanz zu verbessem bei gleichzeitiger Umformung des Haares.

Die Erfindung betrifft daher ein Verfahren zum gleichzeitigen Färben und/öder Farbauffrischen und dauerhaften Verformen von Haaren, bei dem man das Haar mit einem färbenden Verformungsmittel behandelt, erhalten unmittelbar vor dem Gebrauch durch Vermischen einer 6 bis 15 Gew.% eines keratinreduzierenden Stoffs enthaltenden Verformungskomponente (A) mit einer Farbstoffkomponente (B), sodann das färbende Verformungsmittel 3 bis 30 Minuten lang auf das Haar einwirken lässt, das Haar, ohne dass zuvor das Verformungsmittel mit Wasser ausgespült wird, mit einem Fixiermittel behandelt und dieses 1 bis 40 Minuten lang einwirken lasst und schließlich abspult, dadurch gekennzeichnet, dass die Farbstoffkomponente (B), als Oxidationsfarbstoffe mindestens einen oxidativen Kupper sowie mindestens einen oxidativen Farbstoff enthält, der ausgewählt ist aus 4,5-Diamino-1-(2-hydroxyethyl)-1H-pyrazol, 4,5-Diamino-1-(1-methylethyl)-1 H-pyrazol, 4,5-Diamino-1-[(4-methylphenyl)methyl]-1H-pyrazol, 4,5-Diamino-1-[(4-chlorphenyl)methyl]-1H-pyrazol, 4,5-Diamino-1-methyl-pyrazol, 2-Amino-6-methyl-phenol, 2-Amino-5-methyl-phenol sowie p-Aminophenolderivaten der Formel 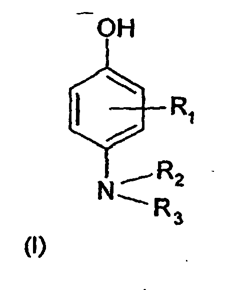 wobei R₁ die Bedeutung H, -CH₃, -CH₂CH₃, -CH₂OH, -CH₂CH₂OH, -CH₂NH₂, -CH₂NHCH₂CH₂OH oder -CH₂OCH₃, hat sowie R₂ und R₃ unabhängig voneinander die Bedeutung H, -CH₃, -CH₂CH₃, -CH₂OH, -CH₂CH₂OH oder -CH₂CH₂CH₂OH haben, unter der Voraussetzung, dass mindestens einer der Reste R₁, R₂ oder R₃ nicht H bedeutet

Vorzugsweise ist der oxidative Farbstoff ausgewählt aus der Gruppe 4,5-Diamino-1-(2-hydroxyethyl)-1H-pyrazol, 4,5-Diamino-1-(1-methylethyl)-1 H-pyrazol, 4,5-Diamino-1-[(4-methylphenyl)methyl]-1H-pyrazol, 4,5-Diamino-1-[(4-chlorphenyl)methyl]-1H-pyrazol, 4,5-Diamino-1-methyl-pyrazol, 2-Amino-6-methyl-phenol, 2-Amino-5-methyl-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol und 4-Amino-2-(methoxymethyl)-phenol, wobei die 4,5-Diamino-pyrazolderivate besonders bevorzugt sind.

Bevorzugt ist der oxidative Kuppler ausgewählt aus der Gruppe 1,3-Diaminobenzol, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methylbenzol, 2,4-Diamino-1-(2-hydroxy- ethoxy)-5-methyl-benzol, 2,4-Di[(2-hydroxyethyl)amino]-1,5-dimethoxy-benzol, 2,4-Diamino-1-(2-hydroxyethoxy)-benzol, 3-Di[(2-hydroxyethyl)amino]-anilin, 4-Amino-1-ethoxy-2-di[(2-hydroxyethyl)amino]-benzol, 3-[(2-Hydroxy-ethyl)amino]-anilin, 3-[(2-Aminoethyl)amino]-anilin, 1,3-Di(2,4-diaminophenoxy)-propan, 2,4-Dimethoxy-1,3-diamino-benzol, 2,6-Bis(2-Hydroxyethyl)amino-toluol, 3-Dimethylaminophenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 3-Diethylamino-phenol, 3-Amino-2-Chlor-6-methyl-phenol, 3-Aminophenol, 3-[(Amidomethyl)amino]-phenol, 5-[(2-Hydroxyethyl)amino]-2-methyl-phenol, 3-[(2-Hydroxyethyl)amino]-phenol, 5-Amino-2-ethyl-phenol, 5-[(3-Hydroxypropyl)amino]-2-methyl-phenol, 3-[(2,3-Dihydroxypropyl)amino]-2-methyl-phenol, 3-[(2-Hydroxy-ethyl)amino]-2-methyl-phenol, 5-Amino-4-chlor-2-methyl-phenol, Methylendioxyphenol, Resorcin, Methylresorcin und 4-Chlorresorcin.

Die Farbstoffkomponente (B) kann zusätzlich eine Oxidationsfarbstoffvorstufe enthalten, welche ausgewählt ist aus 1,4-Diaminobenzol, 1,4-Diamino-2-methylbenzol, 1,4-Diamino-2,6-dimethylbenzol, 1,4-Diamino-2,5-dimethyl-benzol, 1,4-Diamino-2,3-dimethyl-benzol, 1,4-Diamino-2-chlor-benzol, 4-Di[(2-hydroxyethyl)aminol-anilin, 4-[(2-Methoxyethyl)amino]-anilin, 1,4-Diamino-2-(2-hydroxyethyl)-benzol, 1,3-Bis-[N(2-Hydroxyethyl)-N-(4-Aminophenyl)-amino-2-propanol und 2,'2-[1,2-Ethandiyl-Bis(Oxy-2,1-Ethandiyloxy)]Bis-1,4-diaminobenzol.

Die Farbstoffkomponente (B) kann zusätzlich direktziehende Farbstoffe enthalten, welche ausgewählt sind aus folgenden Gruppen:

### Nitrofarbstoffe (blau)

4-N-Ethyl-N-(2'-hydroxyethyl)amino-1-(2'-hydroxyethyl)amino-2-nitro-benzol, 1-Amino-3-methyl-4-(2'-hydroxyethyl)amino-6-nitro-benzol, 1-(2'-Hydroxyethyl)amino-2-nitro-4-bis-(2"-hydroxyethyl)amino-benzol, 4-Bis-(2'-hydroxyethyl)amino-1-(2"-methoxyethyl)amino-nitrobenzol, 1-(2',3'-Dihydroxypropyl) amino-2-nitro-4-[ethyl-(2"-hydroxyethyl)amino]-benzol, 1-[(2',3'-Dihydroxypropyl)amino]-2-nitro-4-[ethyl-2"-(hydroxyethyl)amino]-benzol, 1-(3'-Hydroxypropylamino)-2-nitro-4-bis-(2"-hydroxyethylamino)-benzol.

### Nitrofarbstoffe (rot)

1-Amino-4-(2'-hydroxyethyl)-amino-nitrobenzol, 1-Hydroxy-2-amino-4,6-dinitrobenzol, 1-Amino-2-nitro-4-bis-(2'-hydroxyethyl)amino-benzol, 1-Amino-2-nitro-4-(2'-hydroxyethyl)amino-5-chlorbenzol, 1-(2'Hydroxyethyl)amino-2-nitro-4-aminobenzol, 1-Hydroxy-3-nitro-4-aminobenzol, 1-(2'-Aminoethyl)amino-2-nitro-4-(2"-hydroxyethyl)-oxybenzol, 3-Nitro-4-(2'-hydroxyethyl)amino-phenylglycerinether, 1-Amino-5-chlor-4-(2',3'-dihydroxypropyl)amino-2-nitro-benzol, 1,4-Bis-[(2',3'-dihydroxypropyl)amino-5-chlor-2-nitro-benzol, 1-Hydroxy-2(2'hydroxyethyl)amino-4,6-dinitro-benzol, 2-Amino-6-chlor-4-nitrophenol, 1-Hydroxy-3-nitro-4-(3'-hydroxypropylamino)-benzol, 3-Nitro-4-ethylamino-benzoesäure, 4-Amino-2-nitro-diphenylamino-2-carbonsäure, 2-Chlor-6-ethylamino-4-nitrophenol, 2,5-Diamino-6-nitropyridin, 1,2,3,4-Tetrahydro-6-nitrochinoxalin.

### Nitrofarbstoffe (gelb)

4-(2'-Hydroxyethyl)amino-3-nitro-benzonitril, 4-(2'-Hydroxyethyl)amirio-3-nitro-benzamid, 1-Amino-2-(2'-hydroxyethyl)amino-5-nitro-benzol, 1-Methoxy-2-(2'-hydroxyethyl)amino-5-nitro-benzol, 1-Hydroxy-3-nitro-4-(2'-hydroxyethyl)aminobenzol, 1-Hydroxy-2-amino-3-nitro-benzol, 1-Amino-2-methyl-6-nitro-benzol, 1-(2'-Hydroxyethyl)-oxy-3-methylamino-4-nitrabenzol, 1-Methylamino-2-nitro-5-(2',3'-dihydroxypropyl)-oxybenzol, 1-(2'-Hydroxyethyl)amino-2-hydroxy-4-nitrobenzol, 1-Methoxy-3-(2'-aminoethyl)-amino-4-nitro-benzol, 1-(2'-Ureidoethyl)amino-4-nitro-benzol, 1-(2'-Hydroxyethyl)amino-2-nitro-benzol, 4-(2'-Hydroxyethyl)amino-3-nitrotrifluormethyl-benzol, 2,4-Bis-[N-(2'-hydroxyethyl)amino]-5-ohlor-nitrobenzol, 4-(2',3'-Dihydroxypropyl)amino-3-nitro-trifluormethyl-benzol, 4-(2'-Hydroxymethyl)amino-3-nitro-methylbenzol, 4-(2'-Hydroxyethyl)amino-3-nitro-chlorbenzol,

### Azofarbstoffe

1-(4'-Nitrophenylazo)-2-methyl-4-bis-(2"-hydroxyethyl)amino-benzol, 1-(3'-Nitro-4-amino)-phenylazo-2-hydroxy-7-trimethyl-ammoniumchlorid-naphthalin, 1-(2'-Hydroxy-4'-sulfo-6'-nitro)-naphthylazo-2-hydroxynaphthalin, 1-(4'-Aminophenylazo)-2-methyl-4-bis-[(2'-hydroxyethyl)-amino]-benzol, 5-(4-Dimethylaminophenylazo)-1,4-dimethyl-triazoniumchlorid, 1-(2'-Methoxyphenylazo)-2-hydroxy-7-trimethylammonium-naphthalinchorid, 1-(4'-Aminophenylazo)-2-hydroxy-7-trimethylammonium-naphthalin, 4-(3'-Trimethylammoniumphenylazo)-N-phenyl-3-methyl-pyrazolon (5), 4-Hydroxy-3-[(4'-sulfo-l'-naphthyl)azo]-1-naphthalinsulfonsäure, 1-(4'-Sulfophenylazo)-2-hydroxy-naphthalin, 1-(4'-Sulfophenylazo)-2-hydroxy-6-sulfo-naphthalin, 4-Amino-[4'-bis-(2"-hydroxyethyl)amino-azobenzol, 4-Amino-[4'-bis-(2"-hydroxyethyl)amino]-2'-methyl-azobenzol, 3-(2',6'-Diaminopyridyl-3'-azo)-pyridin, 7-Phenylazo-l-amino-3,6-disulfo-8-hydroxy-naphthalin, 5-Acetylamino-4-hydroxy-3-[(2'-methylphenyl)azo]-2,7-naphthalindisulfonsäure, 2-(2',4'-Dimethylphenylazo)-6-(4"-sulfophenylazo)-1,3-dihydroxybenzol.

### Chinonfarbstoffe

1,4-Bis-(2',3'-dihydroxypropyl)amino-anthrachinon, 1-Methylamino-4-(2'-hydroxyethyl)amino-anthrachinon, 2-(2'-Aminoethyl)amino-anthrachinon, 2-Brom-4,8-diamino-6-(3'-trimethylammonium)-phenylamino-1,5-naphthochinon, 1-(2'-Sulfo-4'-methyl-phenyl)-amino-4-hydroxy-anthrachinon, 1,4-Diamino-anthrachinon, 1-Amino-2-sulfo-4-cyclo-hexylamino-anthrachinon, 1-Methylamino-4-aminopropylamino-anthrachinon, 1-Aminopropylamino-anthrachinon.

### Triphenylmethanfarbstoffe

4',4",4"'-Triamino-3-methyl-triphenylcarboniumchlorid, Bis-(4,4-Diethylominophenyl)-4'-ethylanlino-naphthyl-carboniumchlorid, Bis-(4,4-Dimethylaminophen)-4'-phenyl-amino-naphthyl-carboniurnchlorid und 4,4-Bis-(N-Ethyl-3-sulfobenzyl)-amino-2"-sulfofuchsonium.

Auch die natürlichen Farbstoffe wie Henna, Indigo oder Yuglon sind geeignet.

Die Farbstoffe werden in der Farbstoffkomponente (B) vorzugsweise in einer Menge von 0,5 bis 30 Gewichtsprozent, besonders bevorzugt 1 bis 10 Gewichtsprozent, verwendet.

Bevorzugt enthält die Farbstoffkomponente (B) ebenfalls einen haarkeratinreduzierenden Stoff. Die Menge des haarkeratinreduzierenden Stoffs ist in der Farbstoffkomponente (B) und in dem gebrauchsfertigen färbenden Verformungsmittel bevorzugt 2 bis 20 Gewichtsprozent, besonders bevorzugt 5 bis 20 Gewichtsprozent.

Die Farbstoffe sind im gebrauchsfertigen färbenden Verformungsmittel in einer Menge von 0,1 bis 5 Gewichtsprozent, besonders bevorzugt 0,5 bis 3 Gewichtsprozent, enthalten.

Zum Erhalt des gebrauchsfertigen Verformungsmittels werden die Verformungskomponente (A) und die Farbstoffkomponente (B) bevorzugt im Verhältnis 1:1 bis 10:1 miteinander vermischt.

Bei dem erfindungsgemäßen Verfahren wird das Haar mit dem Fixiermittel behandelt, ohne das färbende Verformungsmittel zuvor mit Wasser aus dem Haar zu spülen.

In einer bevorzugten Ausführungsform des erfindungsgemäßen Verfahrens wird auf das Haar nach der Einwirkungszeit des färbenden Verformungsmittels und vor der Behandlung mit dem Fixiermittel eine saure Zwischenspülung vom pH = 2 bis 6 aufgebracht.

In einer weiteren bevorzugten Ausführungsform des erfindungsgemäßen. Verfahrens wird auf das Haar nach der Einwirkungszeit des färbenden Verformungsmittels und vor der Behandlung mit dem Fixiermittel mit einem sauren Vorfixiermittel, vorzugsweise mit einem Gehalt an 1 bis 4 Gew.% Wasserstoffperoxid, behandelt.

Die Vorteile der vorliegenden Erfindung liegen in der besseren Haltbarkeit im Vergleich zur Farbauffrischung mittels Tönungsmitteln und in der Haarschonung im Vergleich zur Haarverformung und Färbung in zwei aufeinanderfolgenden Schritten. Darüber hinaus wird der Glanz des Haares sichtbar erhöht. Durch das direkte Auftragen der Fixierung, ohne vorheriges Abspülen der Wellflüssigkeit mit Wasser, kommt es zu einem intensiven Farbergebnis. Der gesamte Behanglungsablauf ist haarschonerider im Vergleich zum normalen Behandlungsablauf einer dauerhaften Haarverformung.

Bei dem erfindungsgemäßen Verfahren wird das Haar gewaschen, mit einem Handtuch frottiert, gegebenenfalls mit einem Teil des zuvor hergestellten färbenden Haarverformungsmittels vorgefeuchtet, in einzelne Strähnen aufgeteilt und auf Wickler gewickelt Auf das aufgewickälte Haar wird anschließend eine für die Haarverformung ausreichende Menge des färbenden Haarverformungsmittels, im allgemeinen (mittellanges Haar) etwa 80 g, aufgetragen.

Die bei dem hier beschriebenen Verfahren verwendbaren Verfomugswirkstoffe sind solche auf der Basis üblicher haarkeratinreduzierender Stoffe, wie zum Beispiel Salze der schwefeligen Säure oder bestimmte Mercaptoverbindungen, insbesondere Salze oder Ester von Mercaptocarbonsäuren. Die Verformungskomponente (A) enthält die keratinreduzierenden Verbindungen in den für die Haarverformung üblichen Mengen, beispielsweise die Ammoniumsalze der Thioglykolsäure oder Thiomilchsäure oder aber Cystein, in einer Konzentration von 6 bis 12 Gewichtsprozent. Der pH-Wert der alkalischen Verformungsmittel liegt im allgemeinen bei 7 bis 10, wobei die Einstellung vorzugsweise mit Ammoniak, Monoethanolamin, Ammoniumcarbonat oder Ammoniumhydrogencarbonat erfolgt.

Ist die Verformungskomponente (A) sauer (zum Beispiel auf pH = 6,5 bis 6,9) eingestellt, werden Ester von Mercaptocarbonsäuren, wie beispielsweise Monothioglykolsäureglykolester oder -glycerinester, vorzugsweise aber Mercaptoacetamide oder 2-Mercaptopropionsäureamide, in einer Konzentration von 2 bis 14 Gewichtsprozent; oder aber die Salze der schwefeligen Säure, zum Beispiel Natrium-, Ammonium- oder Monoethanolammoniumsulfit, in einer Konzentration von 3 bis 8 Gewichtsprozent (berechnet als SO₂), verwendet.

Bevorzugt ist die verwendete haarkeratinreduzierende Verbindung das Salz oder das Derivat einer Mercaptocarbonsäure. Besonders bevorzugt ist die keratinreduzierende Verbindung ausgewählt aus Thioglykolsäure, Cystein und Thiomilchsäure oder deren Salzen.

Zur Wirkungssteigerung können der Verformungskbmponente (A) Quell- und Penetrationsstoffe, wie beispielsweise Harnstoff, mehrwertige Alkohole, Ether, Melamin, Alkali- oder Ammoniumthiocyanat, Isopropanol, Imidazolidin-2-on, 2-Pyrrolidön und 1-Methyl-2-pyrrolidon, in einer Konzentration von etwa 0,5 bis 50 Gewichtsprozent, vorzugsweise 2 bis 30 Gewichtsprozent, zugesetzt werden.

Nach einer für die dauerhafte Verformung des Haares ausreichenden Einwirkungszeit, welche je nach Haarbeschaffehheit, dem pH-Wert und der Verformungswirksamkeit des färbenden Verformungsmittels sowie in Abhängigkeit von der Anwendungstemperatur 3 bis 30 Minuten, bevorzugt 2 bis 20 Minuten, beträgt, wird das Haar, ohne dass das Verformungsmittel zuvor mit Wasser ausgespült wird, oxidativ fixiert.

Das Fixiermittel wird, je nach Haarfülle, in einer Menge von etwa 50 bis 200 g verwendet. Für die Fixierung kann jedes beliebige, bisher in Fixiermitteln verwendete Oxidationsmittel verwendet werden. Beispiele für solche Oxidationsmittel sind Kaliumbromat, Natriumbriomat, Natriumperborat, Dehydroascorbinsäure, Hydrogenperoxid und Hamstoffperdxid. Die Konzentration der Oxidationsmittel ist in Abhängigkeit von Anwendungszeit (in der Regel 1 bis 40 Minuten, bevorzugt 5 bis 20 Minuten) und Anwendungstemperatur (25 bis 50 Grad Celsius) unterschiedlich. Normalerweise werden in den wässrigen Fixiermitteln die Oxidationsmittel in einer Konzentration von etwa 0,5 bis 12,0 Gewichtsprozent verwendet. Das Fixiermittel kann selbstverständlich weitere Stoffe, wie zum Beispiel schwache Säuren oder Peroxidstabilisatoren, enthalten.

Sowohl die bei dem erfindungsgemäßen Verfahren verwendeten färbenden Verformungsmittel als auch die Fixiermittel können in Form einer wässrigen Lösung oder einer Emulsion sowie in verdickter Form auf wässriger Basis, insbesondere als Creme, Gel oder Paste, vorliegen.

Besonders bevorzugt liegt die Fixierung in viskoser Form mit einer Konsistenz vor, welche es ermöglicht, das Haar der Kundin im Sitzen und nicht wie gewöhnlich im Waschbecken zu fixieren. In Verbindung mit dem direkten Auftragen der Fixierung ohne Zwischenspülung mit Wasser wird ein optimales Farbergebnis erzielt. Bevorzugt ist das Fixiermittel eine oxidationsmittelhaltige viskose Zubereitung mit einer Viskosität von 100 bis 10.000 mPa • s bei 25 Grad Celsius, wobei eine Viskosität von 300 bis 1000 mPa • s bei 25 Grad Celsius besonders bevorzugt ist. Die Viskositätsangaben beziehen sich auf die Messung mit einem Haake Rotations-Viskosimeter Typ VT 501 bei einer Schergeschwindigkeit von 64,5 pro Sekunde.

Ebenfalls ist es möglich, diese Mittel unter Druck in Aerosoldosen abzufüllen und daraus als Aerosolsthaum zu entnehmen.

Selbstverständlich können sowohl die Fixiermittel als auch das gebrauchsfertige färbende Verformungsmittel alle für derartige Mittel üblichen und bekannten Zusatzstoffe, zum Beispiel Verdickungsmittel, wie beispielsweise Kaolin, Bentonit; Fettsäuren, höhere Fettalkohole, Stärke, Polyacrylsäure, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quartäre Ammoniumsalze, Alkylbetaine, oxethylierte Fettalkohole, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Haarkonditionierungsmittel sowie haarpflegende Bestandteile, wie zum Beispiel Lanolinderivate, Cholesterin, Pantothensäure, Creatin oder Betain, enthalten. Ferner können diesen Mitteln optische Aufheller in Form von Cumarin-, Stilben-, Naphthalimid-, Benzoxazol- oder Styrylderivaten zugesetzt werden. Die erwähnten Bestandteile werden in den für solche Zwecke üblichen Mengen verwendet, zum Beispiel die Netzmittel und Emulgatoren in Konzentrationen von-etwa 0,2 bis 30 Gewichtsprozent, während die Verdickungsmittel in einer Menge von etwa 0,1 bis 25 Gewichtsprozent, bezogen jeweils auf die gebrauchsfertigen Mittel, enthalten sein können.

Anschließend werden die Wickler entfernt, das Fixiermittel mit Wasser aus dem Haar ausgespült und das Haar in üblicher Weise weiterbehandelt. Vorteilhaft wird das Haar im Anschluss an die färbende Dauerverformung zur Wasserwelle gelegt.

Das auf diese Weise dauerverformte Haar besitzt eine Färbung bzw. Farbauffrischung (Farberneuerung) von deutlich längerer Haltbarkeit als bei einer üblichen Tönung oder Färbung. Das erfindungsgemäße Verfahren bietet den Vorteil, dass eine Verformung des Haares und eine Färbung bzw. Farbauffrischung (Farberneuerung) und Glanzverbesserung in einem Arbeitsgang erreicht wird. Hierdurch wird eine schonende und zugleich zeitsparende Behandlung des Haares ermöglicht. Ein weiterer Vorteil ist die längere Haltbarkeit der Farbe im Vergleich zur Anwendung von Tönungsmitteln nach einer Dauerwellbehandlung.

### Beispiele

### Beispiel 1

Ungeschädigtes Haar wird mit einem Shampoo gewaschen, frottiert und auf Wickler mit einem Durchmesser von 8 Millimeter gewickelt. Vor dem Gebrauch werden 40g der nachfolgenden Komponente A und 40g der nachfolgenden Komponente B zu 80g des gebrauchsfertigen färbenden Verformungsmittels vermischt.

### Komponente A

| | |
|---|---|
| 16,0 g | Ammoniumthioglykolat, 70%-ige wäßrige Lösung |
| 1,0 g | Ammoniak, 25%-ige wäßrige Lösung |
| 4,0 g | Ammoniumhydrogencarbonat |
| 1,0 g | mit 35 mol Ethylenoxid oxethyliertes Rizinusöl |
| 0,5 g | Parfümöl |
| ad 100 g | Wasser |

### Komponente B

| | |
|---|---|
| 10,00 g | Ammoniumthioglykolat, 70%-ige wäßrige Lösung |
| 1,00 g | mit 35 mol Ethylenoxid oxethyliertes Rizinusöl |
| 0,20 g | Natriumsulfit |
| 0,10 g | Parfümöl |
| 0,10 g | 3-Aminophenol |
| 0,04 g | Amino-4-[(2-hydroxyethyl)amino]-anisol |
| 0,60 g | Resorcin |
| 0,40 g | p-Toluylendiaminsulfat |
| 0,03 g | 2-Amino-5-methyl-phenol |
| 0,50 g | 4-Amino-3-methyl-phenol |
| 2,00 g | Ethanol |
| ad 100 g | Wasser |

Beide Komponenten weisen, ebenso wie ihr Gemisch, einen pH-Wert von 8,0 auf. Anschließend wird die gebrauchsfertige färbende Dauerwellösung gleichmäßig auf dem gewickelten Haar verteilt. Sodann erfolgt die Einwirkzeit von 20 min unter Anwendung einer Infrarotstrahler-Haube bei einer Temperatur von 40°C. Sodann wird das aufgewickelte Haar mit 80g des folgenden Fixiermittels behandelt.

### Flüssiges Fixiermittel

| | |
|---|---|
| 4,00 g | Wasserstoffperoxid, 50%-ig |
| 0,10 g | Salicylsäure |
| 0,20 g | Dinatriumhydrogenphosphat |
| 0,15 g | o-Phosphorsäure |
| 1,00 g | mit 35 mol Ethylenoxid oxethyliertes Rizinusöl |
| 0,10 g | Vinylpyrrolidon/Styrol-Mischpolymerisat |
| 0,10 g | Parfümöl |
| ad 100 g | Wasser |

Es folgt eine Einwirkungszeit von 15 Minuten. Nach dem Ablauf der Einwirkzeit werden die Wickler abgewickelt und das Haar mit warmem Wasser gründlich ausgespült. Das Haar ist in einem dunklen Goldblond gefärbt und weist schöne glänzende Locken auf. Es schließt sich die gewohnte Erstellung der Frisur mit. Fön und Bürste an.

### Beispiel 2

Oxidativ geschädigtes und dauergewelltes Haar wird mit einem Shampoo gewaschen, frottiert und auf Wickler mit einem Durchmesser von 8 Millimeter gewickelt.. Vor dem Gebrauch werden je 40g nachfolgenden Komponente A und B zu 80g des gebrauchsfertigen färbenden Haarverformungsmittels vermischt.

### Komponente A

| | |
|---|---|
| 10,0 g | Ammoniumthioglykolat, 70%-ige wäßrige Lösung |
| 0,5 g | Ammoniak, 25%-ige wäßrige Lösung |
| 2,0 g | Ammoniumhydrogencarbonat |
| 1,0 g | mit 35 mol Ethylenoxid oxethyliertes Rizinusöl |
| 0,5 g | Parfümöl |
| 2,0 g | Polydimethyl-dialtyl-ammoniumchlorid-Homopolymer (CTFA: POLYQUATERNIUM-6) |
| ad 100 g | Wasser |

### Komponente B

| | |
|---|---|
| 13,00 g | Ammoniumthioglykolat, 70%-ige wäßrige Lösung |
| 1,00 g | mit 10 mol Ethylenoxid oxethylierter Cocosfettalkohol |
| 0,04 g | 1,3-Diamino-4-(2-hydroxyethoxy)-benzol-sulfat |
| 2,00 g | Resorcin |
| 0,30 g | p-Toluylendiaminsulfat |
| 0,80 g | 4-Amino-2-hydroxy-toluol |
| 1,30 g | 4,5-Diamino-1-(2-hydroxyethyl)-1H-pyrazol-sulfat |
| 0,10 g | Parfümöl |
| 3,00 g | Isopropanol |
| ad 100 g | Wasser |

Beide Komponenten A und B weisen einen pH-Wert von 8,0 auf. Anschließend wird die gebrauchsfertige färbende Dauerwellösung gleichmäßig auf dem gewickelten Haar verteilt. Sodann erfolgt die Einwirkzeit von 10 min unter Anwendung einer Infrarotstrahler-Haube bei einer Temperatur von 40°C. Schließlich wird das aufgewickelte Haar mit 80g des folgenden Fixiermittels behandelt.

### Viskoses Fixiermittel

| | |
|---|---|
| 4,00 g | Wasserstoffperoxid, 50%-ige wässrige Lösung |
| 0,50 g | Natriumlaurylsulfat |
| 1,00 g | Dinatriumhydrogenphosphat |
| 4,00 g | Cetylstearylalkohol |
| 0,10 g | Salicylsäure |
| 0,30 g | Parfümöl, |
| ad 100 g | Wasser |

Es folgt eine Einwirkungszeit von 5 Minuten. Nach dem Ablauf der Einwirkungszeit werden die Wickler abgewickelt und das Haar weitere 15 Minuten lang mit der viskosen Fixierung behandelt. Anschließend wird das Haar mit warmem Wasser gründlich ausgespült. Das Haar ist intensiv kupferrot gefärbt und zeigt schöne glänzende Locken. Es schließt sich die gewohnte Erstellung der Frisur mit Fön und Bürste an.

## Patentansprüche

1. Verfahren zum gleichzeitigen Färben und/oder Farbauffrischen und dauerhaften Verformen von Haaren, bei dem man das Haar mit einem färbenden Verformungsmittel behandelt, erhalten unmittelbar vor dem Gebrauch durch Vermischen einer 6 bis 15 Gew.% eines keratinreduzierenden Stoffs enthaltenden färbende Verformungskomponente (A) mit einer Farbstoffkomponente (B), sodann das Verformungsmittel 3 bis 30 Minuten lang auf das Haar einwirken lässt, das Haar, ohne dass zuvor das färbende Verformungsmittel mit Wasser ausgespült wird, mit einem Fixiermittel behandelt und dieses 1 bis 40 Minuten lang einwirken lässt und schließlich abspült, **dadurch gekennzeichnet, dass** die Farbstoffkomponente (B), als Oxidationsfarbstoffe mindestens einen oxidativen Kupper sowie mindestens einen oxidativen Farbstoff enthält, der ausgewählt ist aus 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 4,5-Diamino-1-[(4-chlorphenyl)methyl]-1H-pyrazol, 4,5-Diamino-1-methyl-pyrazol, 2-Amino-6-methyl-phenol, 2-Amino-5-methyl-phenol sowie p-Aminophenolderivaten der Formel 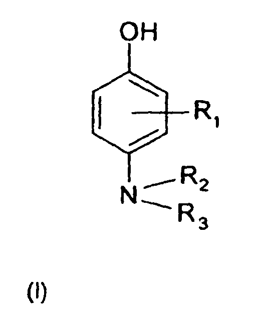 wobei R₁ die Bedeutung H, -CH₃, -CH₂CH₃, -CH₂OH, -CH₂CH₂OH, -CH₂NH₂, -CH₂NHCH₂CH_{Z}OH oder -CH₂OCH₃ hat sowie R₂ und R₃ unabhängig voneinander die Bedeutung H, -CH₃, -CH₂CH₃, -CH₂OH, -CH₂CH₂OH oder -CH₂CH₂CH₂OH haben, unter der Voraussetzung, dass mindestens einer der Reste R₁, R₂ oder R₃ nicht H bedeutet.

2. Verfahren nach Anspruch 1, **dadurch gekennzeichnet, dass** der oxidative Farbstoff ausgewählt ist aus 4,5-Diamino-1-(2-hydroxyethyl)-1H-pyrazol, 4,5-Diamino-1-(1-methylethyl)-1H-pyrazol, 4,5-Diamino-1-[(4-methylphenyl)-methyl]-1H-pyrazol, 4,5-Diamino-1-[(4-chlorphenyl)methyl]-1H-pyrazol, 4,5-Diamino-1-methyl-pyrazol, 2-Amino-6-methyl-phenol, 2-Amino-5-methyl-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol und 4-Amino-2-(methoxymethyl)-phenol.

3. Verfahren nach einem der Ansprüche 1 oder 2, **dadurch gekennzeichnet, dass** der oxidative Kuppler ausgewählt ist aus 1,3-Diamino-benzol, 2-Amino-4-[(2-hydroxyethyl)aminol-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxy- ethoxy)-5-methyl-benzol, 2,4-Di[(2-hydroxyethyl)amino]-1,5-dimethoxy-benzol, 2,4-Diamino-1-(2-hydroxyethoxy)-benzol, 3-Di[(2-hydroxyethyl)amino]-anilin, 4-Amino-1-ethoxy-2-di[(2-hydroxyethyl)amino]-benzol, 3-[(2-Hydroxy-ethyl)amino]-anilin, 3-[(2-Aminoethyl)amino]-anilin, 1,3-Di(2,4 diaminophenoxy)-propan, 2,4-Dimethoxy-1,3-diamino-benzol, 2,6-Bis(2-Hydroxyethyl)amino-toluol, 3-Dimethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 3-Diethylaminophenol, 3-Amino-2-chlor-6-methyl-phenol, 3-Aminophenol, 3 [(Amidomethyl)-amino]-phenol, 5-[(2-Hydroxyethyl)amino]-2-methyl-phenol, 3-[(2-Hydroxyethyl)aminol-phenol, 5-Amino-2-ethyl-phenol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 5-Amino-4-chlor-2-methyl-phenol, Methylendioxyphenol, Resorcin, Methylresorcin und 4-Chlorresorcin.

4. Verfahren nach einem der Ansprüche 1 bis 3 **dadurch gekennzeichnet, dass** es zusätzlich eine Oxidationsfarbstoffvorstufe enthält, ausgewählt aus 1,4-Diaminobenzol, 1,4-Diamino-2-methylbenzol, 1,4-Diamino-2,6-dimethylbenzol, 1,4-Diamino-2,5-dimethyl-benzol, 1,4-Diamino-2,3-dimethyl-benzol, 1,4-Diamino-2-chlor-benzol, 4-Di[(2-hydroxyethyl)amino]-anilin, 4-[(2-Methoxyethyl)amino]-anilin, 1,4-Diamino-2-(2-hydroxyethyl)-benzol, 1,3-Bis-[N(2-Hydroxyethyl)-N-(4-Aminophenyl)-amino-2-propanol und 2,'2-[1,2-Ethandiyl-Bis(Oxy-2,1-Ethandiyloxy)]Bis-1,4-diamino-benzol.

5. Verfahren nach einem der Ansprüche 1 bis 4, **dadurch gekennzeichnet, dass** man die Farbstoffe in der Farbstoffkomponente (B), in einer Menge von 0,5 bis 30 Gewichtsprozent verwendet.

6. Verfahren nach einem der Ansprüche 1 bis 5, **dadurch gekennzeichnet, dass** die Farbstoffe im gebrauchsfertigen färbenden Verformungsmittel in einer Menge von 0,1 bis 5 Gewichtsprozent enthalten sind.

7. Verfahren nach einem der Ansprüche 1 bis 6, **dadurch gekennzeichnet, dass** man die Farbstoffkomponente (B) und die Verformungskomponente (B) im Verhältnis 1/1 bis 1/10 mischt.

8. Verfahren nach einem der Ansprüche 1 bis 7, **dadurch gekennzeichnet, dass** der haarkeratinreduzierende Stoff ein Salz der schwefeligen Säure oder das Salz oder das Derivat einer Mercaptocarbonsäure ist.

9. Verfahren nach Anspruch 8, **dadurch gekennzeichnet, dass** der haarkeratinreduzierende Stoff ausgewählt ist aus Thioglykolsäure, Cystein oder Thiomilchsäure oder deren Salz.

10. Verfahren nach einem der Ansprüche 1 bis 9, **dadurch gekennzeichnet, dass** man den haarkeratinreduzierenden Stoff in dem färbenden Verformungsmittel in einer Menge von 2 bis 20 Gewichtsprozent verwendet.

11. Verfahren nach einem der Ansprüche 1 bis 10, **dadurch gekennzeichnet, dass** die Farbstoffkomponente (B) einen haarkeratinreduzierenden Stoff in einer Menge von 5 bis 20 Gewichtsprozent enthält.

12. Verfahren nach einem der Ansprüche 1 bis 11, **dadurch gekennzeichnet, dass** man als Fixiermittel eine oxidationsmittelhaltige viskose Zubereitung mit einer Viskosität von 100 bis 10.000 mPa · s bei 25 Grad Celsius verwendet.

13. Verfahren nach Anspruch 12, **dadurch gekennzeichnet, dass** man die Viskosität von 300 bis 1000 mPa · s bei 25 Grad Celsius beträgt.

14. Verfahren nach einem der Ansprüche 1 bis 13, **dadurch gekennzeichnet, dass** man auf das Haar nach der Einwirkungszeit des färbenden Verformungsmittels und vor der Behandlung mit dem Fixiermittel eine saure Zwischenspülung vom pH = 2 bis 6 aufbringt.

15. Verfahren nach einem der Ansprüche 1 bis 14, **dadurch gekennzeichnet, dass** man auf das Haar nach der Einwirkungszeit des färbenden Verformungsmittels und vor der Behandlung mit dem Fixiermittel ein saures Vorfixiermittel aufbringt.

## Claims

1. Method of simultaneously dyeing and/or reviving the colour of and permanently waving the hair, in which the hair is treated with a colouring shaping agent obtained directly prior to use by mixing a colouring shaping component (A) comprising 6 to 15% by weight of a keratin-reducing substance with a dye component (B), then allowing the shaping agent to act on the hair for 3 to 30 minutes, treating the hair, without rinsing out the colouring shaping agent with water beforehand, with a neutralizer and allowing this to act for 1 to 40 minutes and finally rinsing, **characterized in that** the dye component (B) comprises, as oxidation dyes, at least one oxidative coupler and at least one oxidative dye which is chosen from 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 4,5-diamino-1-[(4-chlorophenyl)methyl]-1H-pyrazole, 4,5-diamino-1-methylpyrazole, 2-amino-6-methylphenol, 2-amino-5-methylphenol, and p-aminophenol derivatives of the formula 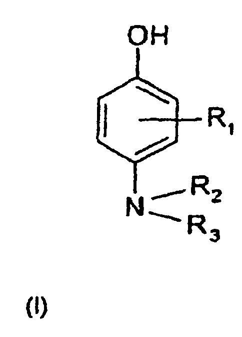 where R₁ has the meaning H, -CH₃, -CH₂CH₃, -CH₂OH, -CH₂CH₂OH, -CH₂NH₂, -CH₂NHCH₂CH₂OH or -CH₂OCH₃, and R₂ and R₃, independently of one another, have the meaning H, -CH₃, -CH₂CH₃, -CH₂OH, -CH₂CH₂OH or -CH₂CH₂CH₂OH, with the prerequisite that at least one of the radicals R₁, R₂ or R₃ is not H.

2. Method according to Claim 1, **characterized in that** the oxidative dye is chosen from 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 4,5-diamino-1-[(4-chlorophenyl)methyl]-1H-pyrazole, 4,5-diamino-1-methylpyrazole, 2-amino-6-methylphenol, 2-amino-5-methylphenol, 4-amino-3-methylphenol, 4-methylaminophenol, 4-amino-2-(aminomethyl)phenol, 4-amino-2-[(2-hydroxyethyl)amino]methylphenol and 4-amino-2-(methoxymethyl)phenol.

3. Method according to one of Claims 1 or 2, **characterized in that** the oxidative coupler is chosen from 1,3-diaminobenzene, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,4-diamino-1-(2-hydroxyethoxy)benzene, 3-di[(2-hydroxyethyl)amino]aniline, 4-amino-1-ethoxy-2-di[(2-hydroxyethyl)amino]benzene, 3-[(2-hydroxyethyl)amino]aniline, 3-[(2-aminoethyl)-amino]aniline, 1,3-di(2,4-diaminophenoxy)propane, 2,4-dimethoxy-1,3-diaminobenzene, 2,6-bis(2-hydroxyethyl)aminotoluene, 3-dimethylaminophenol, 5-amino-2-methylphenol, 5-amino-4-fluoro-2-methyl-phenol, 5-amino-4-methoxy-2-methylphenol, 5-amino-4-ethoxy-2-methylphenol, 3-amino-2,4-dichlorophenol, 3-diethylaminophenol, 3-amino-2-chloro-6-methylphenol, 3-aminophenol, 3-[(amidomethyl)amino]phenol, 5-[(2-hydroxyethyl)amino]-2-methylphenol, 3-[(2-hydroxyethyl)amino]phenol, 5-amino-2-ethylphenol, 5-[(3-hydroxypropyl)amino]-2-methylphenol, 3-[(2,3-dihydroxypropyl)amino]-2-methylphenol, 3-[(2-hydroxyethyl)amino]-2-methyl-phenol, 5-amino-4-chloro-2-methylphenol, methylenedioxyphenol, resorcinol, methylresorcinol and 4-chlororesorcinol.

4. Method according to one of Claims 1 to 3, **characterized in that** it additionally comprises an oxidation dye precursor chosen from 1,4-diaminobenzene, 1,4-diamino-2-methylbenzene, 1,4-diamino-2,6-dimethylbenzene, 1,4-diamino-2,5-dimethylbenzene, 1,4-diamino-2,3-dimethylbenzene, 1,4-diamino-2-chlorobenzene, 4-di[(2-hydroxyethyl)-amino] aniline, 4-[(2-methoxyethyl)amino]aniline, 1,4-diamino-2-(2-hydroxyethyl)benzene, 1,3-bis[N-(2-hydroxyethyl)-N-(4-aminophenyl)amino-2-propanol and 2,2'-[1,2-ethanediylbis(oxy-2,1-ethanediyloxy)]bis-1,4-diaminobenzene.

5. Method according to one of Claims 1 to 4, **characterized in that** the dyes in the dye component (B) are used in an amount of from 0.5 to 30% by weight.

6. Method according to one of Claims 1 to 5, **characterized in that** the dyes are present in the ready-to-use colouring shaping agent in an amount of from 0.1 to 5% by weight.

7. Method according to one of Claims 1 to 6, **characterized in that** the dye component (B) and the shaping component (A) are mixed in the ratio 1/1 to 1/10.

8. Method according to one of Claims 1 to 7, **characterized in that** the hair-keratin-reducing substance is a salt of sulphurous acid or the salt or the derivative of a mercaptocarboxylic acid.

9. Method according to Claim 8, **characterized in that** the hair-keratin-reducing substance is chosen from thioglycolic acid, cysteine or thiolactic acid or salt thereof.

10. Method according to one of Claims 1 to 9, **characterized in that** the hair-keratin-reducing substance in the colouring shaping agent is used in an amount of from 2 to 20% by weight.

11. Method according to one of Claims 1 to 10, **characterized in that** the dye component (B) comprises a hair-keratin-reducing substance in an amount of from 5 to 20% by weight.

12. Method according to one of Claims 1 to 11, **characterized in that** the neutralizer used is a viscous preparation containing oxidizing agent and having a viscosity of from 100 to 10 000 mPa·s at 25 degrees Celsius.

13. Method according to Claim 12, **characterized in that** the viscosity is from 300 to 1000 mPa·s at 25 degrees Celsius.

14. Method according to one of Claims 1 to 13, **characterized in that** an acidic intermediate rinse of pH = 2 to 6 is applied to the hair after the contact time of the colouring shaping agent and before the treatment with the neutralizer.

15. Method according to one of Claims 1 to 14, **characterized in that** an acidic preneutralizer is applied to the hair after the contact time of the colouring shaping agent and before the treatment with the neutralizer.

## Revendications

1. Procédé pour la teinture et/ou l'avivage de la couleur et la mise en forme permanente simultanés des cheveux, dans lequel on traite les cheveux par une composition de mise en forme colorante, obtenue immédiatement avant l'emploi par mélange d'un composant de mise en forme colorant (A), contenant 6 à 15 % en poids d'une substance réduisant la kératine, avec un composant colorant (B), puis on laisse agir pendant 3 à 30 minutes la composition de mise en forme sur les cheveux, on traite les cheveux par un fixateur, sans éliminer au préalable la composition de mise en forme colorante par rinçage avec de l'eau, et on le laisse agir pendant 1 à 40 minutes et enfin on rince les cheveux, **caractérisé en ce que** le composant colorant (B) contient en tant que colorants d'oxydation au moins un coupleur oxydant ainsi qu'au moins un colorant d'oxydation qui est choisi parmi le 4,5-diamino-1-(2-hydroxyéthyl)-1H-pyrazole, le 4,5-diamino-1-(méthyléthyl)-1H-pyrazole, le 4,5-diamino-1-[(4-méthylphényl)-méthyl]-1H-pyrazole, le 4,5-diamino-1-[(4-chlorophényl)méthyl]-1H-pyrazole, le 4,5-diamino-1-méthylpyrazole, le 2-amino-6-méthylphénol, le 2-amino-5-méthylphénol ainsi que des dérivés de p-aminophénol de formule 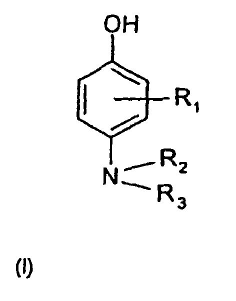 dans laquelle R₁ représente H, -CH₃, -CH₂CH₃, -CH₂OH, -CH₂CH₂OH, -CH₂NH₂, -CH₂NHCH₂CH₂OH ou -CH₂OCH₃ et R₂ et R₃ représentent, indépendamment l'un de l'autre, H, -CH₃, -CH₂CH₃, -CH₂OH, -CH₂CH₂OH, ou -CH₂CH₂CH₂OH, étant entendu qu'au moins l'un des radicaux R₁, R₂ et R₃ ne représente pas H.

2. Procédé selon la revendication 1, **caractérisé en ce que** le colorant d'oxydation est choisi parmi le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl]-1H-pyrazole, le 4,5-diamino-1-[(4-chlorophényl)méthyl]-1H-pyrazole, le 4,5-diamino-1-méthyl-pyrazole, le 2-amino-6-méthyl-phénol, le 2-amino-5-méthyl-phénol, le 4-amino-3-méthyl-phénol, le 4-méthyl-amino-phénol, le 4-amino-2-(aminométhyl)-phénol, le 4-amino-2-[(2-hydroxyéthyl)-amino]méthyl-phénol et le 4-amino-2-(méthoxyméthyl)-phénol.

3. Procédé selon la revendication 1 ou 2, **caractérisé en ce que** le coupleur oxydant est choisi parmi le 1,3-diamino-benzène, le 2-amino-4-[(2-hydroxyéthyl)amino]-anisole, le 2,4-diamino-1-fluoro-5-méthyl-benzène, le 2,4-diamino-1-méthoxy-5-méthyl-benzène, le 2,4-diamino-1-éthoxy-5-méthyl-benzène, le 2,4-diamino-1-(2-hydroxy-éthoxy)-5-méthyl-benzène, le 2,4-di[(2-hydroxyéthyl)amino]-1,5-diméthoxy-benzène, le 2,4-diamino-1-(2-hydroxy-éthoxy)-benzène, la 3-di[(2-hydroxyéthyl)amino]-aniline, le 4-amino-1-éthoxy-2-di [(2-hydroxy-éthyl)amino]-benzène, la 3-[(2-hydroxy-éthyl)-amino]-aniline, la 3-[(2-aminoéthyl)amino]-aniline, le 1,3-di(2,4-diaminophénoxy)-propane, le 2,4-diméthoxy-1,3-diamino-benzène, le 2,6-bis(2-hydroxyéthyl)amino-toluène, le 3-diméthylamino-phénol, le 5-amino-2-méthyl-phénol, le 5-amino-4-fluoro-2-méthyl-phenol, le 5-amino-4-méthoxy-2-méthyl-phénol, le 5-amino-4-éthoxy-2-méthyl-phénol, le 3-amino-2,4-dichlorophénol, le 3-diéthylaminophénol, le 3-amino-2-chloro-6-méthyl-phénol, le 3-aminophénol, le 3-[(amidométhyl)amino]-phénol, le 5-[(2-hydroxyéthyl)amino]-2-méthyl-phénol, le 3-[(2-hydroxyéthyl)amino]-phénol, le 5-amino-2-éthyl-phénol, le 5-[(3-hydroxypropyl)amino]-2-méthyl-phénol, le 3-[(2,3-dihydroxypropyl)amino]-2-méthyl-phénol, le 3-[(2-hydroxyéthyl)amino]-2-méthyl-phénol, le 5-amino-4-chloro-2-méthyl-phénol, le méthylènedioxyphénol, le résorcinol, le méthylrésorcinol et le 4-chlororésorcinol.

4. Procédé selon l'une quelconque des revendications 1 à 3, **caractérisé en ce qu'**il comporte en outre un précurseur de colorant d'oxydation, choisi parmi le 1,4-diaminobenzène, le 1,4-diamino-2-méthylbenzène, le 1,4-diamino-2,6-diméthylbenzène, le 1,4-diamino-2,5-diméthyl-benzène, le 1,4-diamino-2,3-diméthyl-benzène, le 1,4-diamino-2-chlorobenzène, la 4-di[(2-hydroxyethyl)amino]-aniline, la 4-[(2-méthoxyéthyl)amino]aniline, le 1,4-diamino-2-(2-hydroxyéthyl)-benzène, le 1,3-bis[N(2-hydroxyéthyl)-N-(4-aminophényl)-amino-2-propanol et le 2',2-[1,2-éthanediyl-bis(oxy-2,1-éthanediyloxy)]-bis-1,4-diamino-benzène.

5. Procédé selon l'une quelconque des revendications 1 à 4, **caractérisé en ce que** dans le composant colorant (B) on utilise les colorants en une quantité de 0,5 bis 30 % en poids.

6. Procédé selon l'une quelconque des revendications 1 à 5, **caractérisé en ce que** les colorants sont contenus en une quantité de 0,1 à 5 % en poids dans la composition de mise en forme colorante prête à l'emploi.

7. Procédé selon l'une quelconque des revendications 1 à 6, **caractérisé en ce qu'**on mélange le composant colorant (B) et le composant de mise en forme (A) en un rapport de 1:1 à 1:10.

8. Procédé selon l'une quelconque des revendications 1 à 7, **caractérisé en ce que** la substance réduisant la kératine est un sel de l'acide sulfureux ou le sel ou le dérivé d'un acide mercaptocarboxylique.

9. Procédé selon la revendication 8, **caractérisé en ce que** la substance réduisant la kératine est choisie parmi l'acide thioglycolique, la cystéine et l'acide thiolactique et leurs sels.

10. Procédé selon l'une quelconque des revendications 1 à 9, **caractérisé en ce que** la substance réduisant la kératine est utilisée dans la composition de mise en forme colorante en une quantité de 2 à 20 % en poids.

11. Procédé selon l'une quelconque des revendications 1 à 10, **caractérisé en ce que** le composant colorant (B) contient une substance réduisant la kératine en une quantité de 5 à 20 % en poids.

12. Procédé selon l'une quelconque des revendications 1 à 11, **caractérisé en ce qu'**on utilise comme fixateur une préparation visqueuse contenant un oxydant, ayant une viscosité de 100 à 10 000 mPa.s à 25°C.

13. Procédé selon la revendication 12, **caractérisé en ce que** la viscosité va de 300 à 1 000 mPa·s à 25°C.

14. Procédé selon l'une quelconque des revendications 1 à 13, **caractérisé en ce qu'**après le temps d'action de la composition de mise en forme colorante et avant le traitement par le fixateur on applique sur les cheveux une composition acide de rinçage intermédiaire ayant un pH de 2 à 6.

15. Procédé selon l'une quelconque des revendications 1 à 14, **caractérisé en ce que** qu'après le temps d'action de la composition de mise en forme colorante et avant le traitement par le fixateur on applique sur les cheveux une composition acide de préfixage.
